# EUROPEAN PATENT SPECIFICATION

(11) **EP 3 772 318 B1**
(45) Date of publication and mention of the grant of the patent: **30.07.2025**
(21) Application number: 19197903.8
(22) Date of filing: 18.09.2019
(51) Int. Cl.: A61B 1/00, A61B 1/002, A61B 1/015, A61B 1/303, A61B 1/018

(54) **HYSTEROSCOPE**
HYSTEROSKOP
HYSTÉROSCOPE

(30) Priority: 06.08.2019 DE 102019121269
(43) Date of publication of application: 10.02.2021
(73) Proprietor: Delmont Imaging SAS, 13600 La Ciotat (FR)
(72) Inventor: Montillot, Pierre, 13600 La Ciotat (FR); Schulz, Dieter, 78570 Mühlheim a.D. (DE); Brikow, Eugen, 88630 Pfullendorf (DE); Buck, Bernd, 88636 Illmensee (DE)
(74) Representative: Gauche, Raynald André

(56) References cited:
- DE-A1- 3 616 193
- US-A- 5 486 155
- US-A1- 2018 125 567
- US-B1- 6 358 200

## Description

The present invention relates to hysteroscopic systems, and, particularly, to a hysteroscopic instrument, which combines different traditional resectoscopy surgical capabilities, by combining and integrating them into an endoscope that also enables gynecologic mechanical and electro-surgical instruments to be used. Usually it is connected with a light source and an irrigation system. It can be operated by one hand and is typically equipped with an optical unit with an offset eyepiece, a rigid operating channel which enables the insertion of mechanical instruments and electrodes that are both monopolar and bipolar. It continually guarantees a continuous irrigation flow and consequently a clear operating field.

For that purpose the hysteroscope is inserted into the cavum uteri which has to be inflated for inspection or for any surgical procedures. For inflation a special liquid or a gas has to be conveyed through the hysteroscope sheath and, after the inflation has been performed, the pressure in the cavum uteri has to be kept on a defined level.

Typical surgical procedures include endocavitary pathologies such as uterine sessile or pedunculated polyps which are resected with monopolar or bipolar electrodes.

Hysteroscopes are well known state of the art. Early CH 295527 relates to an endoscope and in particular to a rectoscope, hysteroscope, bronchoscope and similar medical instruments, with a tube to be inserted into the body opening and a lighting device for illuminating the parts of the body opening surrounding the end of the tube.

DE OS 2 318 860 describes a hysteroscope, suitable for use with a cervical adaptor comprising a barrel for yielding a viewing system and having an inlet passage for feeding insufflation gas into the barrel, for spreading apart the walls of the uterus, and means for holding the uterine walls in their spread apart condition when the introduction of insufflation gas is discontinued. This spreading devices include a plurality of spreader arms which are biased away from each other. They are carried on an annular member which can axially slide in the barrel between a retracted position in which the arms are displaced towards each other by the distal end of the barrel and a spread-out position in which the arms, having been freed from the restraint of the distal end of the barrel and are displaced away from each other and contact the uterine walls. Furthermore an inlet for a probe may be provided. The spreader arms may be in the form of closed loops.

DE 20 02 829 C2 relates to a hysteroscope of the type comprising an outer envelope forming a cannula, means for insufflation of a fluid, particularly a gaseous fluid, such as for example carbon dioxide, inside the uterine cavity distending the walls thereof, and an inner envelope forming an endoscope tube, of smaller diameter than the outer envelope and adapted to slide therein, this inner envelope containing light transmission means for conducting light to the distal end of said inner envelope and comprising on the one hand means for placing mechanically the hysteroscope in operating position, substantially fluid-tight at the level of the cervix of the uterus and on the other hand a portable device for supplying said fluid, fast with said hysteroscope.

US 5,320,091 A shows a continuous flow hysteroscope. This hysteroscope includes an outer sheath having an outer surface, an inner surface and a hollowed-out central area extending along a central axis which extends there through. The outer sheath comprises a proximal section and a distal section. The distal end includes a plurality of openings communicating between the outer surface and inner surface. The openings have a predetermined shape and pass fluid exterior to the distal section therethrough and into the hollowed-out central area. The proximal section of the outer sheath includes an outlet and an inlet. An inner member is positioned within the hollowed central area and includes a first channel which is adapted to receive a telescope. A second channel, which is substantially parallel to the first channel is utilized for passing a working tool. The first and second channel are operatively coupled to an inlet for passing fluid there through and out of the distal end outer sheath. The fluid passageway cooperates with the plurality of openings to pass fluid through the opening from the outer surfaces to the inner surfaces, through the fluid passageway and through the outlet of the outer sheath. The inner member includes an obturator closure device to isolate inflow and outflow distally, to prevent cross flow and to reduce patient trauma at time of insertion of the hysteroscope.

EP 1 325 704 B1 describes a hysteroscope with a shank exchange system. It has an optics housing provided at a proximal end, with an outer shank releasably connected to the optics housing, and with an inner shank which is arranged inside and parallel to the outer shank and is releasably connected to the optics housing. A corresponding endoscope set and a set of endoscope shanks are also provided.

WO 2011/150111 A1 shows an endoscope with a hollow outer sheath configured for insertion into a patient's body. A hollow inner sheath is receivable within the outer sheath, and is configured to slideably receive a first internal instrument. A working element can be configured to rotatably support at least the first internal instrament and a second internal instrument such that at least the first internal instrument and the second internal instrument are rotatable around a common axis of rotation being substantially coextensive with a longitudinal axis of the outer sheath. The working element can have at least one guide rail extending longitudinally of the axis of rotation and an actuator block can be slideably mountable to the at least one guide rail and so securable to at least one of the instruments as to be able to push the at least one of the internal instruments longitudinally of the outer sheath.

WO 2012/044705 A1 describes a hysteroscopic system which includes a scope having an internal channel, a sheath removably coupled to the scope, and an outflow channel. The sheath has a distal flange extending internally towards an outer surface of the scope. The outflow channel is formed between an inner surface of the sheath and an outer surface of the scope. The distal flange forms a distal end of the outflow channel and is generally located between the scope and the sheath.

WO 2017/035221 A2 shows a sheath adapter that may be used within a medical device to provide one or more additional lumens. One sheath for use with a medical device may comprise a C-shaped elongate shaft extending from a proximal end to a distal end, the C-shaped elongate shaft configured to fit onto a shaft of the medical device and a lumen extending at least partially along a length of the elongate shaft, the lumen having a first port disposed proximate the distal end of the C-shaped elongate shaft and a second port disposed proximate the proximal end of the C-shaped elongate shaft.

US 6 358 200 B1 discloses a continuous flow endoscope according to the preamble of claim 1, the endoscope comprising a working element with a frame, the frame including a telescope guide tube having a varying cross- sectional shape along its length and a fluid passage through the frame and into the telescope guide tube; and an outer sheath assembly connected to the frame, the outer sheath assembly comprising an outer tube located around a portion of the telescope guide tube, wherein the outer tube and the telescope guide tube form a fluid outflow conduit there between along a majority of the length of the outer tube. The endoscope may have a telescope extending through the telescope guide tube, wherein the telescope and the guide tube form a fluid inflow conduit there between, and the telescope guide tube may have a first cross-sectional shape along a majority of a length of the guide tube and a second different cross-sectional shape at a front end section of the guide tube. The working element could comprise a connector for directly connecting the working element to the outer sheath assembly and the connector could be rotatable such that the outer sheath assembly can rotate relative to the guide tube. US 5 486 155 A and US 2018/125567 A1 disclose similar endoscopes.

Despite the fact that this endoscopic technology is known for a long time and its use is very common, some serious problems concerning the irrigation could not yet be solved sufficiently. On the one hand the valves of the hysteroscope connecting it with the inlet flow and with the outlet flow have to be very tight but easy going at the same time and the handling should be very easy despite the fact that the hoses filled with pressurized fluid are heavy, bulky, unwieldy and sometimes even inflexible. Those hoses therefore apply unwanted force and momentum to the connections of the hysteroscope and impede the operating person, especially when moving the position.

During usage of the hysteroscope the doctor shouold be able to change instruments without releasing the pressure of the irrigation and without losing the inflating effect of the uterus. The operator has to resect, grasp, extract respectively remove material while irrigation is still running continuously. The connection of the irrigation system should allow to turn the hysteroscope about a longitudinal axis by one hand while the other hand of the surgeon is coping with the devices and exchanging these instruments for different operation steps. During the surgical procedure the surgeon needs his view of the operating field respective for the visualization system of the endoscope and no devices of the irrigation system should distract his attention.

The most advanced technology to solve that functionality is the endoscopic system of HEOS, Hysteroscopy Endo-operative System, as described by Acc. Dr. R. Ricciardi, "HEOS - In respect of women, Sopro Comeg 2017". It solves many of these requirements by a hysteroscopic system, comprising a straight forward rigid hysteroscope, having a main body with parallel eyepiece, a light source adapter for connecting a light source, a tube having a distal end with a distal window, this tube having a fiber optic light transmission incorporated and a rod lenses optical system, and being equipped with at least one working channel and an irrigation system with separate channels for inflowing and outflowing fluid, as well as a hysteroscopy sheath connecting the tube and the main body.

But cleaning and sterilization of that instrument is expensive and difficult. The instrument is equipped with a multitude of valves. All valves do have small dead spaces and tiny dead volumes where infectious particles can accumulate during operation. All bearings which allow axial turning of the scope are potential subject of contamination and all sealings could leak. The smaller the diameters of the whole hysteroscope are, the better for the patient, but the worse for the solidity and the tightness of the instrument. Deformation in the micrometer scale as a result of mechanical stress leads to small leakings. Unfortunately these little deformations disappear during sterilization, as little openings are closing with mechanical stress releasing, and captured microbes can survive. This problem is the reason why many parts of that instrument are designed as spare parts for one-time use and which have to be replaced for every new use, after having been removed and disposed of before sterilization.

The task of the invention therefore is, to improve this hysteroscopic system in a way that these disadvantages are avoided.

The solution is a hysteroscopic system, comprising the features of claim 1.

According to the invention, the rotatable irrigation system can remain at a fixed position during the surgical operation while the rest of the hysteroscope can be turned and rotated so that the distal window can be directed to different viewing fields. It also means that instruments can be inserted into the working channel while the irrigation is still running and the uterus remains pressurised and inflated. This is due to a sophisticated quick lock system.

Besides, even the instrument connection end piece can be rotated in that way that the stopcock and the stopcock ventilation can be adjusted in a position comfortable to the surgeon. For that purpose the end piece is designed to comprise a screw sleeve, a stopcock and stopcock ventilation which are assembled in a way that the screw sleeve is attached to the screw thread of the main body adapter which is connected to the main body.

Optionally an additional working channel can be provided similar to the state of the art at the main body adapter. This can be equipped with a stopcock and it can be used to insert a flexible instrument into the working channel or to provide additional irrigation if necessary.

The drawings briefly described below show the invention in more detail without limiting it to the examples shown:
- Fig. 1:: A sketch of the hysteroscope from the left side,
- Fig. 2:: A perspective sketch of the hysteroscope from front left side,
- Fig. 3:: A sketch of the hysteroscope from the right side,
- Fig. 4:: A perspective sketch of the hysteroscope from front right side,
- Fig. 5:: A sectional drawing of the quick lock from above,
- Fig. 6:: A perspective cut of the outer tube at the quick lock,
- Fig. 7a, 7b:: A cut through the plane of the quick lock push button,
- Fig. 8:: A sketch of the irrigation tube from above,
- Fig. 9:: A perspective sketch of the irrigation connection from front left side,
- Fig. 10:: A perspective cut through the irrigation flow supply
- Fig. 11:: A sectional drawing of the irrigation flow supply,
- Fig. 12:: A sketch of the hysteroscope from the left side with irrigation system,
- Fig. 13:: A perspective sketch of the hysteroscope from back left side with irrigation system.

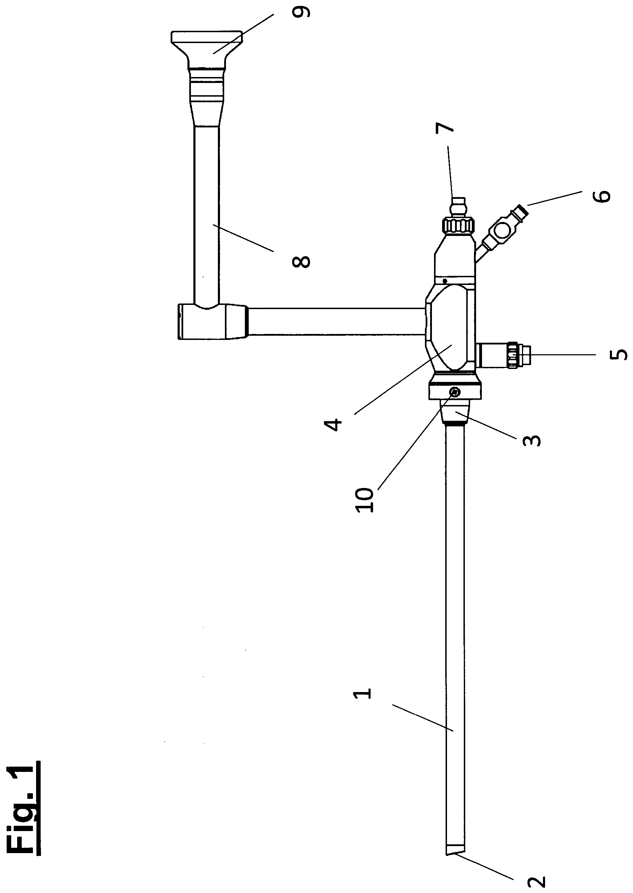

Fig. 1 shows a sketch of the hysteroscope as it is seen from the left hand side of the operator. The outer tube 1 has a distal opening 2 with a distal window, an illumination means, optical means and at least one working channel for instruments and irrigation. The outer tube 1 is connected with the main body 4, the main body 4 is also connected to the quick lock adapter 3 and to the connection for illumination fibre 5. The hysteroscope is equipped with a connection for instruments 7 and an additional working channel 6, which is optional. An optic rod 8 lense is arranged parallel to the main working channel to allow the operator to use the instruments while looking into the eyepiece 9 and to observe the surgical work at the same time.

Fig. 1 does not show the irrigation system nor the surgical instruments. But for connecting the irrigations system the quick lock fixation 10 is shown.

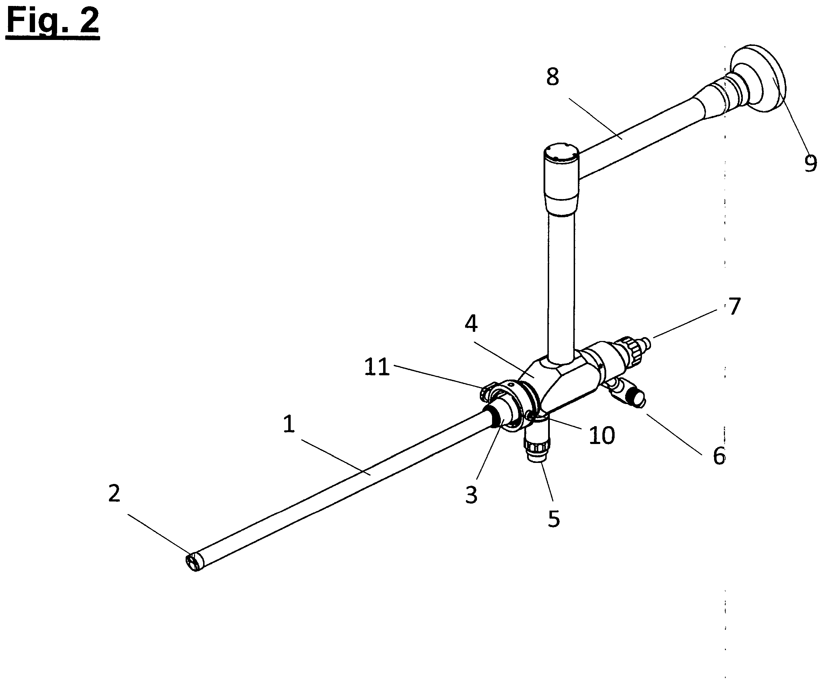

Fig. 2 shows the same object as Fig. 1 in as perspective sketch, as it is seen from the front of the left hand side of the operator. On the opposite side of the quick lock fixation 10 the quick lock lever 11 can be seen.

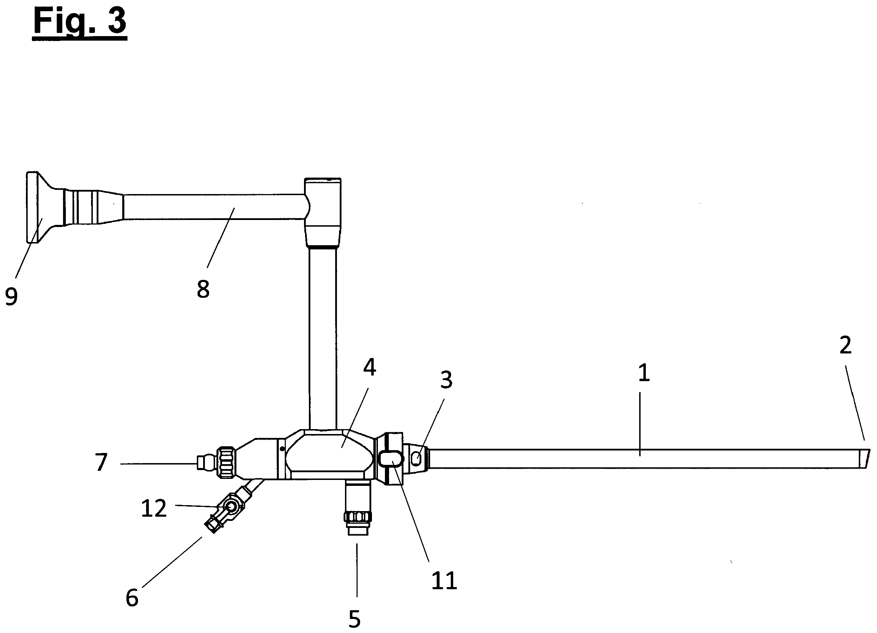

Fig. 3 shows a sketch of the same object from the right hand side of the operator.

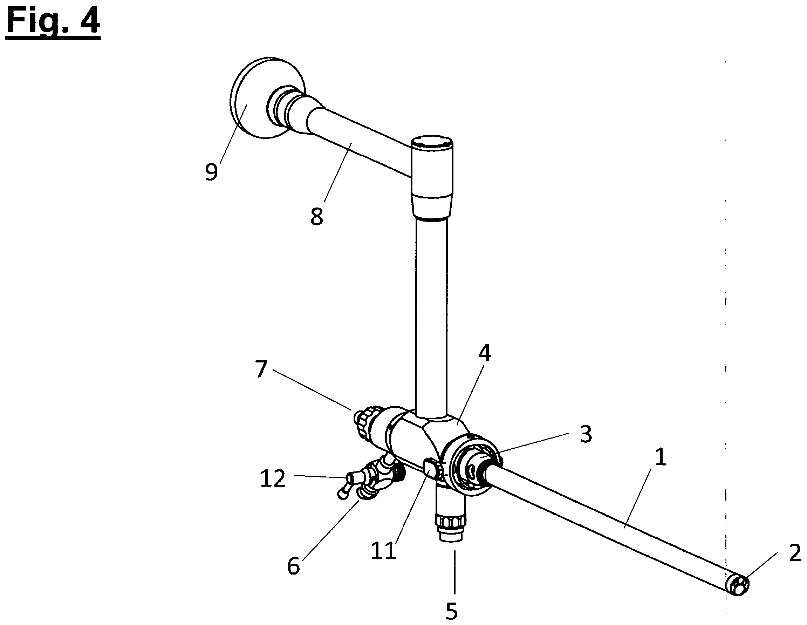

Fig. 4 shows the same object from the front of the right hand side of the operator, from this view the stopcock 12 of the additional working channel 6 can be seen.

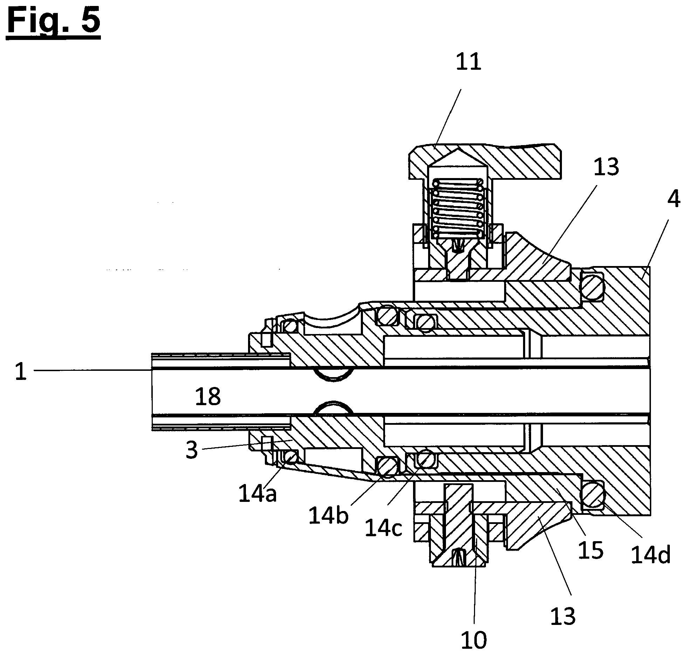

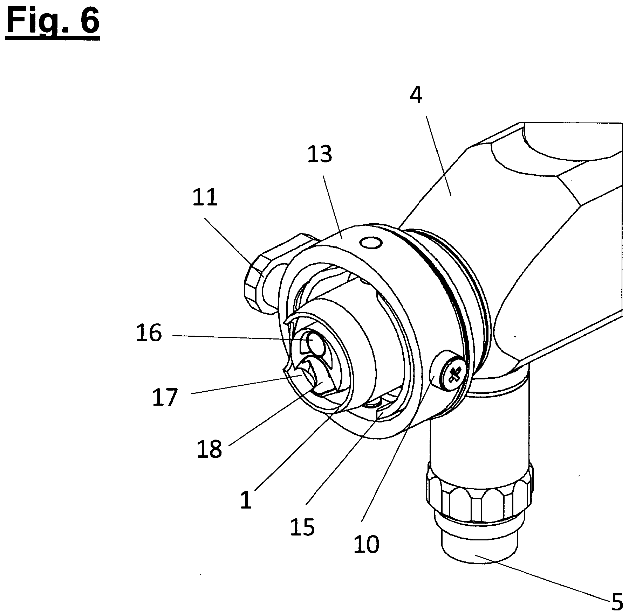

Fig. 5 shows a sectional drawing of the quick lock seen from above. The quick lock adapter 3 is assembled to the main body 4, the lock for the outer shaft 13 and the second quick lock adapter 15. These assembled parts are sealed by the sealings 14a, 14b, 14c, and 14d. Fig. 6 shows the same object as Fig. 5 from a perspective cut of the outer tube at the quick lock. The tube 1 contains the optical system 16, the working channel 18 and an annular space 17 which is reserved for the returning irrigation fluid.

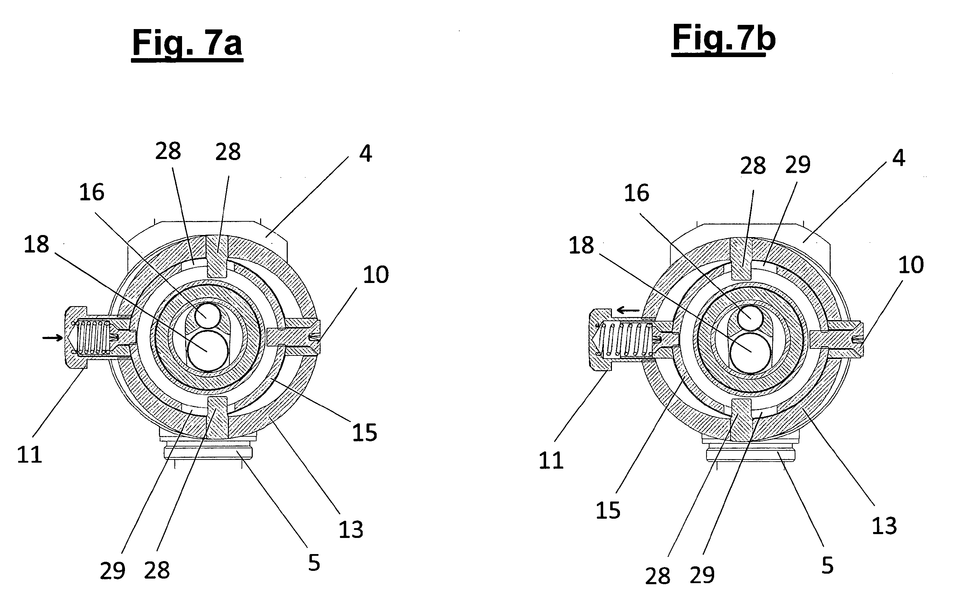

Figs. 7a and 7b show the quick lock system. Fig. 7a shows a cut through the plane of the quick lock button 11 in the moment when it is pressed down for release of the irrigation system. Pressing down causes the lock for the outer shaft 13 moving to right side so that the quick lock pins 28 are moved along the pin-receptive openings 29. Fig. 7b shows the moment when the push button 11 is pressed again and the lock for the outer shaft 13 moves back. During operation it moves back after the irrigation system is assembled.

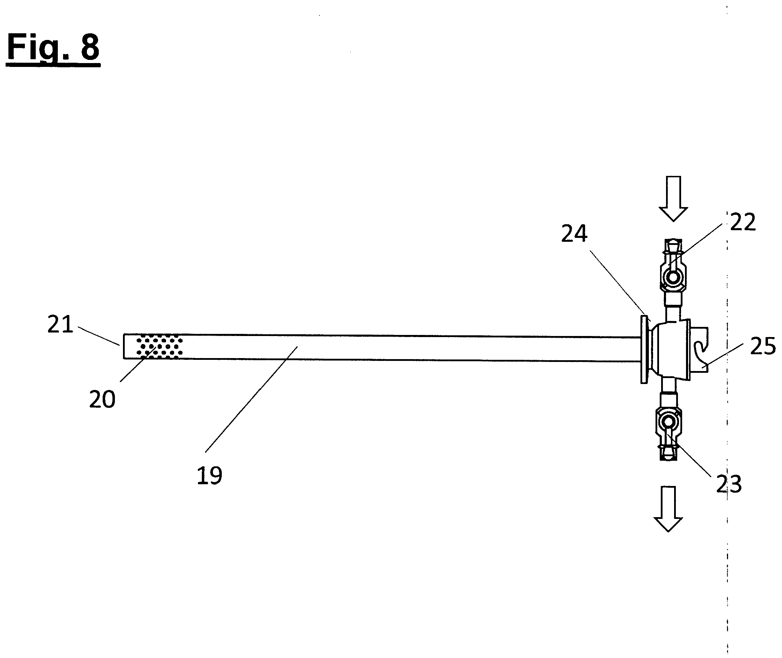

Fig. 8 shows a sketch of the irrigation tube from above, with the outer shaft 19, the distal irrigation inlet 20 and the distal irrigation outlet 21. The irrigation fluid is provided through the irrigation stopcock inlet side 22 and returns after usage to the irrigation stopcock outlet side 23. The distribution of the fluid is performed by the quick lock irrigation distributor 24.

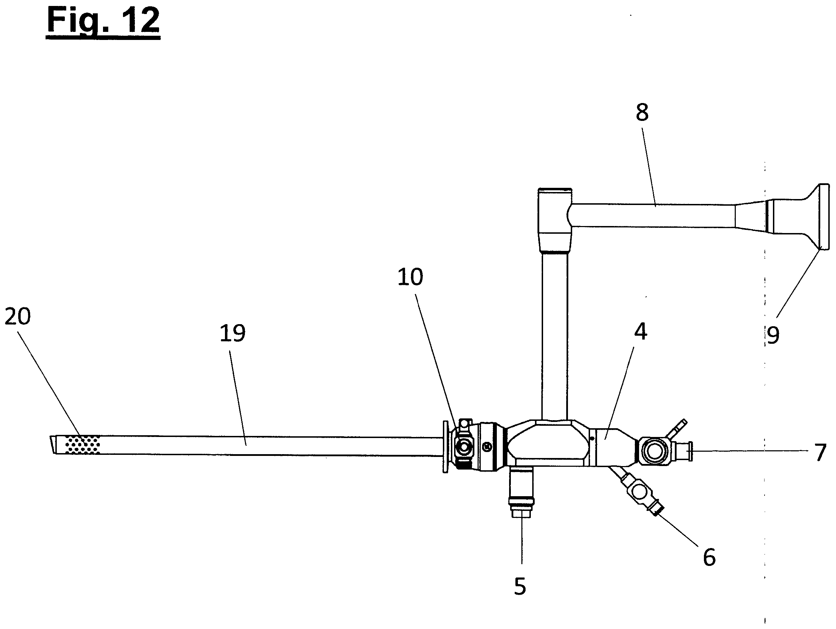

The locking of the irrigation system is performed by pushing the quick lock closing mechanism 25 into the joint quick lock adapters 3 and 15 as shown in Figs. 7a and 7b, where the quick lock pins 28 receive the quick lock closing mechanism after sticking it into the pin-receptive openings 29, the irrigation system is thereby being pulled into the quick lock adapter. For unlocking the push button 11 is pushed and the irrigation system is removed from the quick lock adapters 15 as described in Figs. 12a and 12b.

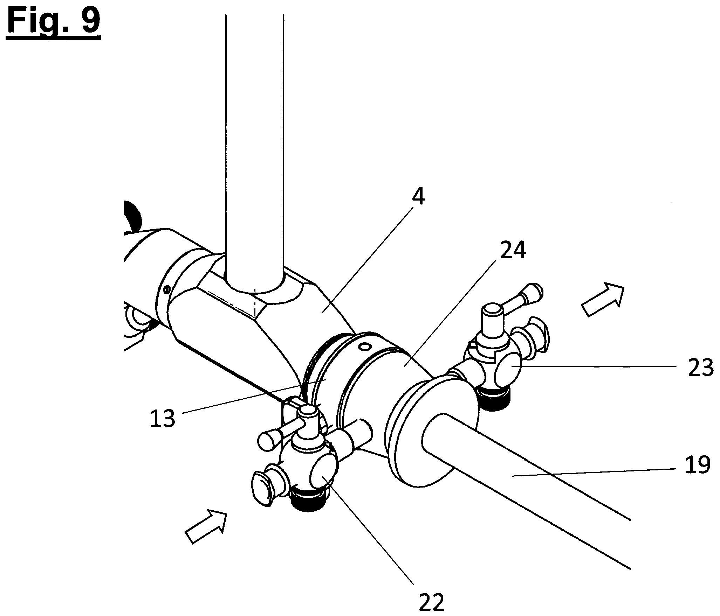

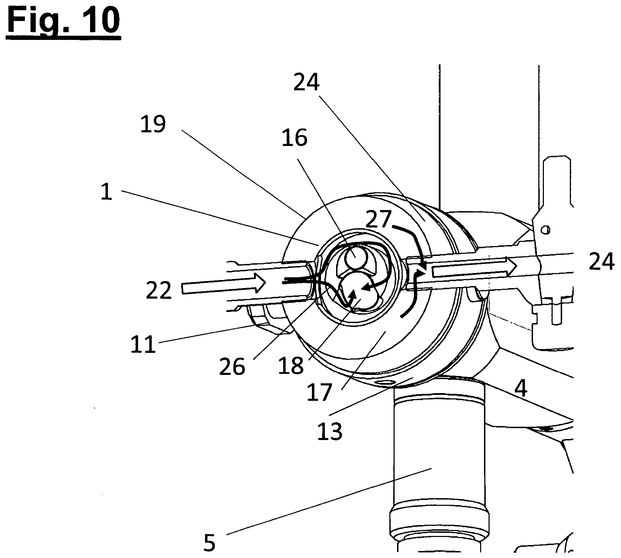

Fig. 9 shows a perspective sketch of the irrigation connection from the front left side. Fig. 10 shows a perspective cut through the irrigation flow supply. The irrigation fluid 22 is provided to the working channel 18. As the inner tube 1 is rotatable the path of irrigation fluid depends on the rotational status of the inner tube, but as two paths exists as shown by the indicating arrows 26, they balance each other so that any rotational status has the same irrigation supply at any time. The returning irrigation fluid uses the outer tube and is drawn of via the pathway 27 as indicated by the arrows to the outlet 23.

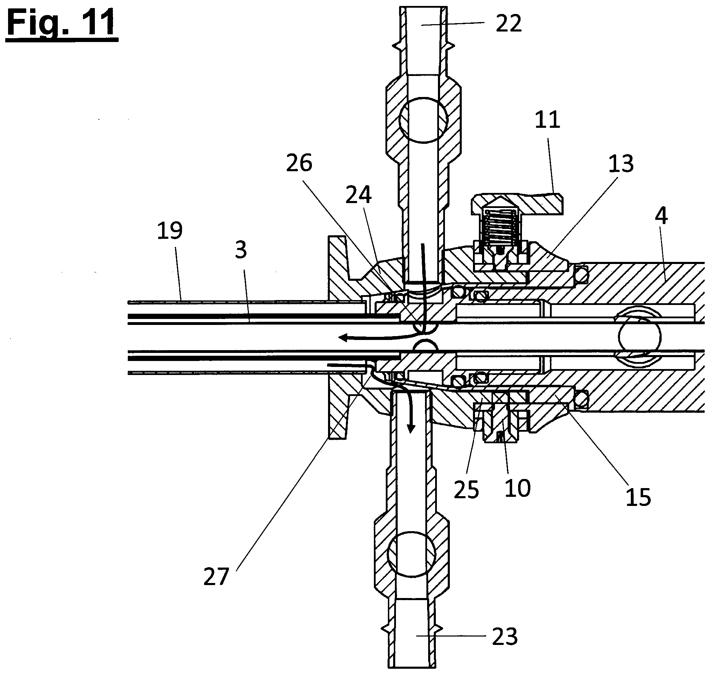

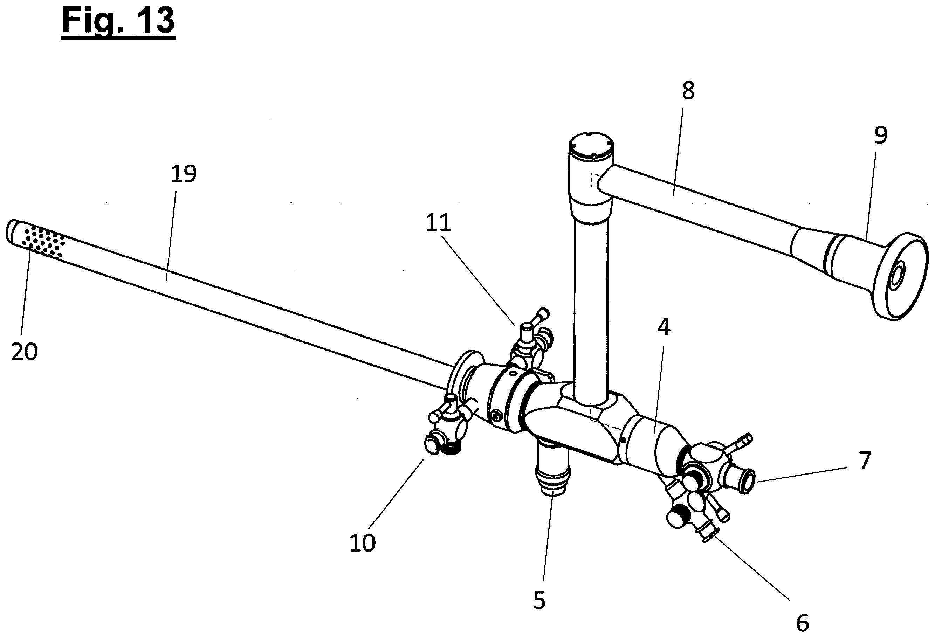

Fig. 11 shows the same object as a sectional drawing of the irrigation flow supply. One can see the locking mechanism 25 with the joint quick lock adapters 3 and 15 and the irrigations paths 26 and 27 as indicated by arrows. Figs. 12 and 13 show the hysteroscope after mounting the irrigation system with the outer tube 19.

### Reference list

- 1: Outer tube
- 2: Distal opening
- 3: Quick lock adapter
- 4: Main body
- 5: Connection for illumination fibre
- 6: Additional working channel
- 7: Connection for instruments
- 8: Fibre optic rod
- 9: Eyepiece
- 10: Quick lock fixation
- 11: Quick lock push button
- 12: Additional working channel stopcock
- 13: Lock for outer shaft
- 14a, 14b,14c,14d: Mechanical seal/ mechanical bearing
- 15: Quick lock adapter
- 16: Optical system
- 17: Annular volume
- 18: Working channel
- 19: Outer shaft
- 20: Distal irrigation inlet
- 21: Distal irrigation outlet
- 22: Irrigation stopcock inlet side
- 23: Irrigation stopcock outlet side
- 24: Quick lock irrigation distributor
- 25: Quick lock closing mechanism
- 26: Irrigation flow inlet path
- 27: Irrigation flow outlet path
- 28: Quick lock pin
- 29: Pin-receptive opening

## Claims

1. Hysteroscopic system, comprising
• a straight forward rigid hysteroscope, having
• a main body (4) with a parallel eyepiece (9), a light source adapter (5) for connecting a light source,
• a tube (1) having an outer shaft (19), a distal end (2) with a distal window,
• that tube (1) having a fiber optic light transmission and an optical system with rod lenses incorporated, and equipped with at least one working channel,
• that tube (1) fixed with the main body (4),
• an axially rotatable irrigation system with separate channels for inflowing and outflowing fluid, the irrigation system comprising a quick lock irrigation distributor (24) configured to perform distribution of the fluid,
• the main body (4) of that hysteroscope being connected to the axially rotatable irrigation system by means of a quick lock system where the rotation of the irrigation system does not interfere with the position of the tube (1) of the hysteroscope, its working channel (18), its fiber optic light transmission system, its optical system (16), and its main body (4), and
• means for irrigating fluid at the distal end of the tube, wherein the quick lock system comprises
• at least two quick lock adapters (3, 15) being of annular cross-section shape, the inner one of them (3) in touch with the tube and the outer one (15) in touch with the main body (4), and both of them assembled together,
• **characterised in that** the quick lock system further comprises a lock for the outer shaft (13), having a greater diameter than the outer one of the at least two quick lock adapters (15) and assembled in touch with the outer quick lock adapter (15),
• a quick lock push button (11) to move the lock for the outer shaft (13) to a side,
• a quick lock fixation (10) as guidance for the movement of the lock for the outer shaft (13),
• at least two pin-receptive openings (29) in the outer one of the quick lock adapters (15),
• quick-lock pins (28) movable along the pin-receptive openings (29), the quick-lock pins (28) being assembled to the lock for the outer shaft (13), wherein pressing down the quick lock button (11) causes the lock for the outer shaft (13) to move to a side so that the quick lock pins (28) are moved along the pin-receptive openings (29), a quick lock closing mechanism (25) of the quick lock irrigation distributor, placed adjacent to the lock for the outer shaft (13), and
• a cavity in the quick lock closing mechanism (25) which fits exactly with the quick lock pins (28) assembled to the lock for the outer shaft (13), such that a locking of the irrigation system is performed by pushing the quick lock closing mechanism (25) into the two joint quick lock adapters (3, 15) whereby the quick lock pins (28) receive the quick lock closing mechanism (25).

## Patentansprüche

1. Hysteroskopisches System, umfassend
• ein gerades starres Hysteroskop, aufweisend
• einen Hauptkörper (4) mit einem Parallelokular (9), einen Lichtquellenadapter (5) zum Anschluss einer Lichtquelle,
• einen Schlauch (1) mit einem Außenschaft (19), einem distalen Ende (2) mit einem distalen Fenster,
• wobei der Schlauch (1) eine faseroptische Lichtübertragung und ein optisches System mit integrierten Stablinsen aufweist und mit mindestens einem Arbeitskanal ausgestattet ist,
• wobei der Schlauch (1) mit dem Hauptkörper (4) befestigt ist,
• ein axial drehbares Bewässerungssystem mit getrennten Kanälen für einströmende und ausströmende Flüssigkeit, wobei das Bewässerungssystem einen Schnellverschluss-Bewässerungsverteiler (24) umfasst, der so eingerichtet ist, dass er die Verteilung der Flüssigkeit durchführt,
• wobei der Hauptkörper (4) dieses Hysteroskops mittels eines Schnellverschlusssystems mit dem axial drehbaren Bewässerungssystem verbunden ist, wobei die Drehung des Bewässerungssystems die Position des Schlauchs (1) des Hysteroskops, seines Arbeitskanals (18), seines faseroptischen Lichtübertragungssystems, seines optischen Systems (16) und seines Hauptkörpers (4) nicht beeinträchtigt, und
• Mittel zum Bewässern von Flüssigkeit am distalen Ende des Schlauchs,
wobei das Schnellverschlusssystem Folgendes umfasst:
• mindestens zwei Schnellverschlussadapter (3, 15) ringförmiger Querschnittsform, wobei der innere (3) mit dem Schlauch in Berührung kommt und der äußere (15) mit dem Hauptkörper (4) in Berührung kommt, und beide zusammen montiert sind,
• **dadurch gekennzeichnet, dass** das Schnellverschlusssystem 2 ferner einen Verschluss für den äußeren Schaft (13) umfasst, der einen größeren Durchmesser aufweist als der äußere der mindestens zwei Schnellverschlussadapter (15) und mit dem äußeren Schnellverschlussadapter (15) in Berührung kommend montiert ist,
• einen Schnellverschlussdruckknopf (11), um den Verschluss für den Außenschaft (13) zur Seite zu bewegen,
• eine Schnellverschlussfixierung (10) als Führung für die Bewegung des Verschlusses für den Außenschaft (13),
• mindestens zwei Stiftaufnahmeöffnungen (29) in dem äußeren der Schnellverschlussadapter (15),
• Schnellverschlussstifte (28), die entlang der Stiftaufnahmeöffnungen (29) beweglich sind, wobei die Schnellverschlussstifte (28) mit dem Verschluss für den Außenschaft (13) verbunden sind, wobei das Herunterdrücken des Schnellverschlussknopfes (11) den Verschluss für den Außenschaft (13) zur Seite bewegt, sodass die Schnellverschlussstifte (28) entlang der Stiftaufnahmeöffnungen (29) bewegt werden, ein Schnellverschluss-Schließmechanismus (25) des Schnellverschluss-Bewässerungsverteilers, der neben dem Verschluss für den Außenschaft (13) platziert ist, und
• einen Hohlraum im Schnellverschluss-Schließmechanismus (25), der genau zu den Schnellverschlussstiften (28) passt, die mit dem Verschluss für den Außenschaft (13) montiert sind, so dass ein Verschluss des Bewässerungssystems durch Drücken des Schnellverschluss-Schließmechanismus (25) in die beiden gemeinsamen Schnellverschlussadaptern (3, 15) erfolgt, wobei die Schnellverschlussstifte (28) den Schnellverschluss-Schließmechanismus (25) aufnehmen.

## Revendications

1. Système hystéroscopique, comprenant
• un hystéroscope rigide droit, présentant
• un corps principal (4) avec un oculaire parallèle (9), un adaptateur de source lumineuse (5) pour connecter une source lumineuse,
• un tube (1) présentant une tige externe (19), une extrémité distale (2) avec une fenêtre distale
• ce tube (1) présentant une transmission de lumière par fibre optique et un système optique avec des lentilles à tige incorporées, et étant équipé d'au moins un canal de travail,
• ce tube (1) étant fixé avec le corps principal (4),
• un système d'irrigation rotatif axialement avec des canaux séparés pour l'entrée et la sortie de fluide, le système d'irrigation comprenant un distributeur d'irrigation à verrouillage rapide (24) configuré pour effectuer la distribution du fluide,
• le corps principal (4) de cet hystéroscope étant connecté au système d'irrigation rotatif axialement au moyen d'un système à verrouillage rapide où la rotation du système d'irrigation n'interfère pas avec la position du tube (1) de l'hystéroscope, de son canal de travail (18), de son système de transmission de lumière par fibre optique, de son système optique (16) et de son corps principal (4), et
• des moyens pour irriguer le fluide à l'extrémité distale du tube,
dans lequel le système à verrouillage rapide comprend
• au moins deux adaptateurs à verrouillage rapide (3, 15) de forme de section transversale annulaire, l'un d'entre eux interne (3) en contact avec le tube et l'un externe (15) en contact avec le corps principal (4), et les deux assemblés ensemble,
• **caractérisé en ce que** le système à verrouillage rapide comprend en outre un verrou pour l'arbre externe (13), présentant un diamètre supérieur à l'un externe des au moins deux adaptateurs à verrouillage rapide (15) et assemblé en contact avec l'adaptateur à verrouillage rapide externe (15),
• un bouton-poussoir à verrouillage rapide (11) pour déplacer le verrou pour l'arbre externe (13) vers un côté,
• une fixation à verrouillage rapide (10) comme guidage pour le mouvement du verrou pour l'arbre externe (13),
• au moins deux ouvertures réceptrices de goupilles (29) dans l'un externe des adaptateurs à verrouillage rapide (15),
• des goupilles à verrouillage rapide (28) mobiles le long des ouvertures réceptrices de goupilles (29), les goupilles à verrouillage rapide (28) étant assemblées au verrou pour l'arbre externe (13), dans lequel l'enfoncement du bouton à verrouillage rapide (11) amène le verrou pour l'arbre externe (13) à se déplacer vers un côté de sorte que les goupilles à verrouillage rapide (28) soient déplacées le long des ouvertures réceptrices de goupilles (29), un mécanisme de fermeture à verrouillage rapide (25) du distributeur d'irrigation à verrouillage rapide, placé adjacent au verrou pour l'arbre externe (13), et
• une cavité dans le mécanisme de fermeture à verrouillage rapide (25) qui s'adapte exactement aux goupilles à verrouillage rapide (28) assemblées au verrou pour l'arbre externe (13), de sorte qu'un verrouillage du système **d'irrigation** est effectué en poussant le mécanisme de fermeture à verrouillage rapide (25) dans les deux adaptateurs à verrouillage rapide (3, 15) joints moyennant quoi les goupilles à verrouillage rapide (28) reçoivent le mécanisme de fermeture à verrouillage rapide (25).
